# FASCICULE DE BREVET EUROPEEN

(11) **EP 0 882 708 B1**
(45) Date de publication et mention de la délivrance du brevet: **22.08.2001**
(21) Numéro de dépôt: 98401030.6
(22) Date de dépôt: 28.04.1998
(51) Int. Cl.: C07C 315/06, C07C 317/04

(54) **Procédé de purification de diméthylsulfoxyde (DMSO)**
Verfahren zur Reinigung von Dimethylsulfoxid (DMSO)
Process for the purification of dimethylsulphoxide (DMSO)

(30) Priorité: 15.05.1997 FR 9705965
(43) Date de publication de la demande: 09.12.1998
(73) Titulaire: ELF AQUITAINE EXPLORATION PRODUCTION FRANCE, 92400 Courbevoie (FR)
(72) Inventeur: Commarieu, Annie, 92400 Courbevoie (FR); Humblot, Francis, 64300 Lanneplaa (FR)
(74) Mandataire: Leboulenger, Jean

(56) Documents cités:
- FR-A- 2 014 385
- A.M. PHIPPS: "Anion exchange in simethyl sulphoxide" ANALYTICAL CHEMISTRY, vol. 40, no. 12, octobre 1968, pages 1769-1773, XP000563242 WASHINGTON, DC, US
- T. CHAUDRON, ET AL.: "La purification du diméthylsulfoxyde: critères de pureté" CHIMIE ANALYTIQUE, vol. 53, no. 5, mai 1971, pages 310-314, XP002004909 PARIS, FR
- KIRK-OTHMER: "Encyclopedia of chemical technology, 4th Edition, Vol. 14, page 778", 1992, WILEY INTERSCIENCE,

## Description

La présente invention concerne un procédé de purification de diméthylsulfoxyde (DMSO).

Le DMSO disponible actuellement sur le marché est un produit ayant déjà une bonne pureté. Ses spécifications commerciales sont généralement :

| | |
|---|---|
| pureté | ≥ 99,7 % par chromatographie |
| acidité | ≤ 0,04 mg KOH/g par potentiométrie |
| point de cristallisation | ≤ 18,1°C |
| aspect visuel | ≤ limpide |
| teneur en eau | ≤ 0,15 % |
| couleur (APHA) | ≤ 10 |

Le brevet FR 2 014 385 décrit un procédé de préparation de DMSO purifié mettant en oeuvre un échangeur d'ions. Dans les deux exemples de ce brevet, on utilise une résine fortement basique du type Amberlite IR-A 400 ou Merck III, pour traiter des mélanges ternaires diméthylsulfure/DMSO/acide sulfurique à 10 %. En fait, dans ce procédé connu, la purification semble essentiellement apportée par une distillation fractionnée d'une solution aqueuse de DMSO traitée auparavant par un échangeur d'anions.

On a effectué maintenant des analyses de traces métalliques sur plusieurs échantillons de DMSO commercial, de différentes provenances. Ces analyses sont rapportées dans le tableau 1.

Les concentrations en sodium, fer, potassium, calcium, chrome, cuivre, nickel et zinc ont été mesurées par ICP (spectrométrie d'émission atomique-torche à plasma, appareil Perkin Elmer, modèle Optima 3000) et sont exprimées en ppb (1 ppb = 1 partie en poids par milliard = 1 µg par kg).

La liste des éléments métalliques figurant dans le tableau 1 n'est pas exhaustive quant aux éléments métalliques présents dans ces échantillons.

**TABLEAU 1**

| Echantillon | Cations métalliques | | | | | | | |
|---|---|---|---|---|---|---|---|---|
| | Na | Fe | K | Ca | Cr | Cu | Ni | Zn |
| 1 | 40 | 13 | 60 | 20 | 2 | 10 | 8 | 10 |
| 2 | 39 | 60 | 3 | 13 | 13 | < 2 | 18 | 3 |
| 3 | 30 | 40 | 3 | 20 | 12 | < 2 | 15 | 3 |
| 4 | 30 | 40 | 3 | 14 | 13 | < 2 | 15 | 3 |
| 5 | 30 | < 1 | 20 | 25 | < 2 | < 2 | < 3 | < 3 |
| 6 | 70 | 90 | 65 | 55 | 15 | 2 | 25 | 60 |
| limite de détection | 2 | 1 | 3 | 2 | 2 | 2 | 3 | 3 |

Pour certaines applications, comme par exemple en électronique ou en pharmacie, les DMSO analysés ci-dessus contiennent trop d'impuretés métalliques. En général, un DMSO contenant moins de 10 ppb de chaque contaminant métallique, alcalin et alcalino-terreux, serait nécessaire pour la plupart des utilisations dans les deux domaines techniques précités.

Le but de la présente invention est de trouver un procédé de purification du DMSO commercial ayant déjà une bonne pureté, mais cette dernière étant cependant insuffisante pour certaines applications.

L'échange d'ions par la mise en oeuvre de résines est une technique de purification connue (Kirk-Othmer, Encyclopedia of Chemical Technology , vol.14, page 778 4th ed. 1992). L'échange d'anions en milieu DMSO liquide à faible teneur en eau a déjà été réalisé par Alan M. Phipps, Anal. Chem. 40(12) pp. 1769-1773, 1968, dans le but de mesurer les quantités d'anions fixés sur la résine dans des conditions expérimentales s'approchant de l'équilibre thermodynamique.

Il a maintenant été trouvé que l'utilisation d'une résine sous forme sulfonate d'ammonium permet, dans du DMSO à faible teneur en eau ou quasi-anhydre, de remplacer les cations métalliques Mⁿ⁺ (n valant de 1 à 4) par des ions ammonium (n.NH₄⁺).

L'invention a donc pour objet un procédé de purification de diméthylsulfoxyde pour en diminuer la teneur en cations métalliques, alcalins et alcalino-terreux, caractérisé en ce qu'il consiste essentiellement à mettre le DMSO à purifier en contact avec un solide constitué par une résine échangeuse d'ions de type sulfonique ayant ses groupes actifs sous forme sulfonate d'ammonium (SO₃NH₄), puis à séparer du solide le liquide constitué par du DMSO purifié à de très faibles teneurs en cations métalliques, alcalins et alcalino-terreux, par tout moyen connu approprié, notamment filtration, percolation ou centrifugation.

Selon l'invention, on traite un DMSO à faible teneur en eau, cette teneur étant inférieure ou égale à 0,15 % en poids par rapport au poids total.

Selon l'invention, la résine sulfonique est à base d'un copolymère polystyrène-divinylbenzène. En effet, ces résines ont un squelette résistant aux attaques chimiques et, en particulier, elles ne se dissolvent pas dans le DMSO. Ces résines sont généralement définies par leur taux de divinylbenzène. En effet, ce dernier détermine le taux de réticulation de la résine et donc la taille des pores dans lesquels l'échange cationique se fait à l'échelle atomique.

Selon l'invention, le divinylbenzène représente de 50 à 60 % en poids et le polystyrène de 50 à 40 % en poids par rapport au poids total du copolymère, sans tenir compte des groupes SO₃NH₄. Ce taux de divinylbenzène assure une bonne activité cinétique de l'échange des cations Mⁿ⁺ par n.NH₄⁺.

La mise en contact du DMSO à purifier avec la résine échangeuse d'ions a lieu à une température allant de 18,45°C (point de fusion du DMSO) à 120°C (température limite de stabilité thermique des résines). Cette température est avantageusement comprise entre 19 et 80°C, de préférence entre 20 et 50°C.

Pour définir la qualité du DMSO susceptible d'être obtenu purifié par le procédé selon l'invention, le fer et le sodium ont été retenus comme éléments traceurs et indicateurs de la teneur générale en cations métalliques, alcalins et alcalino-terreux.

Ce DMSO purifié se caractérise en ce qu'il comporte une teneur en cation Fe inférieure ou égale à 1 ppb et une teneur en cation Na inférieure ou égale à 2 ppb, limites respectives de détection de la méthode d'analyse par spectrométrie d'émission atomique-torche à plasma.

L'invention sera mieux comprise à l'aide de la partie expérimentale suivante décrivant un exemple de réalisation de la présente invention.

### Partie expérimentale

### I. Méthode d'analyse :

Pour analyser les traces de métaux dans le DMSO, on a utilisé l'ICP (spectrométrie d'émission atomique-torche à plasma) : l'échantillon est introduit dans une torche plasma, les différents éléments présents sont excités et émettent des photons dont l'énergie est caractéristique de l'élément puisqu'elle est définie par la structure électronique de l'élément considéré. On a utilisé en routine un appareil Perkin Elmer (modèle Optima 3000 DV).

### II. Méthodologie :

Principe : les traces métalliques sont sous forme Mⁿ⁺. Par passage du DMSO sur une résine échangeuse de cations, elle-même sous forme NH₄⁺, on substitue les ions Mⁿ⁺ en solution par n.NH₄⁺.

### III. Essai :

Principe : par souci de simplifier les analyses, le sodium et le fer ont été choisis comme traceurs représentatifs de l'ensemble des impuretés métalliques contenues dans le DMSO.

Le sodium est caractéristique de la pollution atmosphérique et accidentelle (poussières, environnement) et le fer est caractéristique de la pollution pouvant provenir du process (unité en acier inox).

Du DMSO dopé à 1000 ppb de fer et 1000 ppb de sodium a été mis au contact d'une résine échangeuse de cations, sous forme NH₄⁺ (2 g de résine pour 100 g de DMSO) à 25°C. Des échantillons de DMSO sont prélevés au cours du temps. On peut ainsi suivre les évolutions des concentrations en fer et sodium avec le temps.

La résine employée était de type sulfonique, fournie par la société Purolite sous la référence MN 500. Elle est notamment caractérisée par un rapport divinyl-benzène/styrène compris entre 50/50 et 60/40. Elle a été traitée au préalable pour obtenir la forme H⁺ de la manière suivante : au travers de 90 ml de résine, placés dans une colonne, on fait passer 540 ml d'HCI 5 % à un débit constant et tel que l'opération dure 30 à 45 minutes. La résine a ensuite été rincée à l'eau déionisée jusqu'à neutralité de l'eau sortante. La résine ainsi obtenue sous forme H⁺ a ensuite été traitée pour obtenir la forme NH₄⁺ de la manière suivante : sur les 90 ml de résine, placés dans la colonne, on a fait passer 500 ml d'une solution aqueuse d'ammoniaque (NH₄OH) à 2,5 % à un débit constant et tel que l'opération dure 30 à 45 minutes. Après avoir été rincée à l'eau déminéralisée jusqu'à neutralité de l'eau sortante, la résine a été séchée par mise en suspension dans du méthanol et évaporation sous vide à l'évaporateur rotatif (90°C, 2000 Pa) jusqu'à observation d'un poids constant.

Le tableau 2 indique l'évolution en fonction du temps de la concentration en fer et en sodium dans le DMSO.

**TABLEAU 2**

| | | | | | | | | |
|---|---|---|---|---|---|---|---|---|
| Temps (min.) | 0 | 5 | 10 | 20 | 45 | 60 | 90 | 120 |
| [Na](ppb) | 980 | 120 | 50 | 21 | 10 | 6 | <2 | < 2 |
| [Fe](ppb) | 1020 | 350 | 150 | 70 | 25 | 15 | 6 | < 1 |

## Revendications

1. Procédé de purification d'un diméthylsulfoxyde (DMSO) ayant une teneur en eau inférieure ou égale à 0,15 % en poids par rapport au poids total , caractérisé en ce qu'il consiste à mettre le DMSO à purifier en contact avec un solide constitué par une résine échangeuse d'ions de type sulfonique ayant ses groupes actifs sous forme sulfonate d'ammonium (SO₃NH₄), cette résine étant à base d'un copolymère polystyrène-divinylbenzène dans lequel le divinylbenzène représente de 50 à 60% en poids du copolymère, puis à séparer du solide le liquide constitué par du DMSO purifié.

2. Procédé selon la revendication 1, caractérisé en ce que la mise en contact du DMSO à purifier avec la résine échangeuse d'ions a lieu à une température de 19 à 80°C.

3. Procédé selon la revendication 2, caractérisé en ce que la température est comprise entre 20 et 50°C.

## Patentansprüche

1. Verfahren zur Reinigung eines Dimethylsulfoxids (DMSO) mit einem Wassergehalt kleiner oder gleich 0,15 Gew.-%, bezogen auf das Gesamtgewicht, dadurch gekennzeichnet, daß es darin besteht, das zu reinigende DMSO mit einem Feststoff zusammenzubringen, der aus einem Ionenaustauscherharz des Sulfontyps besteht, dessen aktive Gruppen in Form von Ammonium-sulfonat (SO₃NH₄) vorliegen, wobei diesem Harz ein Polystyrol-Divinylbenzol-Copolymeres zugrundeliegt, in dem das Divinylbenzol 50 bis 60 Gew.-% des Copolymeren beträgt, dann die aus gereinigtem DMSO bestehende Flüssigkeit vom Feststoff zu trennen.

2. Verfahren nach Anspruch 1, dadurch gekennzeichnet, daß das Zusammenbringen des zu reinigenden DMSO mit dem Ionenaustauscherharz bei einer Temperatur von 19 bis 80 °C stattfindet.

3. Verfahren nach Anspruch 2, dadurch gekennzeichnet, daß die Temperatur zwischen 20 und 50 °C liegt.

## Claims

1. Process for purifying a dimethyl sulphoxide (DMSO) having a water content of less than or equal to 0.15% by weight relative to the total weight, characterized in that it consists in placing the DMSO to be purified in contact with a solid consisting of an ion-exchange resin of sulphonic type containing its active groups in the form of an ammonium sulphonate (NH₄SO₃), this resin being based on a polystyrene-divinylbenzene copolymer in which the divinylbenzene represents from 50% to 60% by weight of the copolymer, and then in separating from the solid the liquid consisting of purified DMSO.

2. Process according to Claim 1, characterized in that the placing in contact of the DMSO to be purified with the ion exchange. resin takes place at a temperature of from 19 to 80°C.

3. Process according to Claim 2, characterized in that the temperature is from 20 to 50°C.
